(19) Europäisches Patentamt
European Patent Office
Office européen des brevets

(11) Publication number: 0 359 505 A1

(12) # EUROPEAN PATENT APPLICATION

(21) Application number: 89309202.3

(22) Date of filing: 11.09.89

(51) Int. Cl.[5]: C 07 D 401/06
A 61 K 31/44

(30) Priority: 12.09.88 US 243387

(43) Date of publication of application:
21.03.90 Bulletin 90/12

(84) Designated Contracting States:
AT BE CH DE ES FR GB GR IT LI LU NL SE

(71) Applicant: SMITHKLINE BEECHAM CORPORATION
One Franklin Plaza
Philadelphia Pennsylvania 19103 (US)

(72) Inventor: Kruse, Lawrence Ivan
9 Great Valley Parkway
Malvern, PA 19355 (US)

Finkelstein, Joseph Alan
7549 Brentwood Road
Philadelphia Pennsylvania 19151 (US)

(74) Representative: Giddings, Peter John, Dr. et al
Smith Kline & French Laboratories Ltd. Corporate
Patents Mundells
Welwyn Garden City Hertfordshire AL7 1EY (GB)

Claims for the following Contracting States: ES + GR.

(54) **Dopamine-beta hydroxylase inhibitors.**

(57) Dopamine-β-hydroxylase inhibitors of structure:

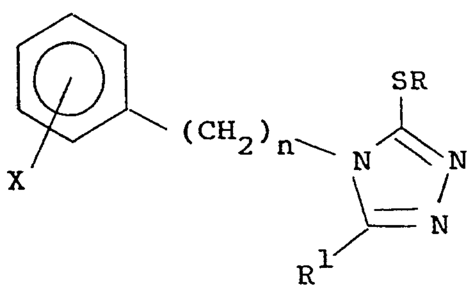

in which $R^1$ is pyridyl or pyridylalkyl, pharmaceutical compositions containing them and their use in therapy in lowering blood pressure.

EP 0 359 505 A1

Description

# DOPAMINE-β-HYDROXYLASE INHIBITORS

## FIELD OF THE INVENTION

This invention relates to novel compounds that inhibit dopamine-β-hydroxylase.

## BACKGROUND OF THE INVENTION

In the catecholamine biosynthetic pathway, tyrosine is converted in three steps to norepinephrine (NE). Intermediates are dihydroxyphenylalanine (DOPA) and dopamine (DA). Dopamine is hydroxylated to norepinephrine by dopamine-β-hydroxylase (DBH) in the presence of oxygen and ascorbic acid.

Inhibition of catecholamine activity decreases blood pressure. Weinshilboum, Mayo Clin. Proc. 55, 39 (1980), reviews compounds that inhibit catecholamine activity by acting upon adrenergic receptors. Alternatively, the catecholamine biosynthetic pathway can be suppressed at any of the three steps, resulting in reduced NE levels. In addition to producing an antihypertensive effect, inhibitors of NE synthesis are active as diuretics, natriuretics, cardiotonics, and vasodilators. Inhibition of DBH activity can have the added advantage of increasing DA levels, which as reported by Ehrreich et al., "New Antihypertensive Drugs," Spectrum Publishing, 1976, pp.409-432, has selective vasodilator activity at certain concentrations.

DBH inhibitors also have been shown to reduce or prevent formation of gastric ulcers in rats by Hidaka et al., "Catecholamine and Stress," edit. by Usdin et al., Permagin Press, Oxford, 1976, pp.159-165 and by Osumi et al., Japan J. Pharmacol. 23, 904 (1973).

A number of DBH inhibitors are known. These generally are divided into two classes, namely, metal chelating agents, which bind copper in the enzyme, and phenethylalamine analogues. Rosenberg et al., "Essays in Neurochemistry and Neuropharmacology," Vol. 4, ed. by Youdim et al., John Wiley & Sons, 1980, pp. 179-192, and Goldstein, Pharmacol. Ref. 18(1), 77 (1966), review DBH inhibitors.

Known DBH inhibitors include:

(a) 5-alkylpicolinic acid [See, Suda et al., Chem. Pharm. Bull. 17, 2377 (1969); Umezawa et al., Biochem. Pharmacol. 19, 35 (1969); Hidaka et al., Mol. Pharmacol. 9, 172 (1973); Miyano et al., Chem. Pharm. Bull. 26, 2328 (1978); Miyano et al., Heterocycles 14, 755 (1980); Claxton et al., Eur. J. Pharmacol. 37, 179 (1976)];

(b) BRL 8242 [see Claxton et al., Eur. J. Pharmacol. 37, 179 (1976)];

(c) 1-alkylimidazole-2-thiols [See, Hanlon et al., Life Sci. 12, 417 (1973); Fuller et al., Adv. Enzyme Regul. 15, 267 (1976)];

(d) substituted thioureas [See, Johnson et al., J. Pharmacol. Exp. Ther. 168, 229 (1969)]; and

(e) benzyloxyamine and benzylhydrazine [See, Creveling et al., Biochim. Biophys. Acta 64, 125 (1962); Creveling et al., Biochim. Biophys. Acta 8, 215 (1962); Van Der Schoot et al., J. Pharmacol. Exp. Ther. 141, 74 (1963); Bloom, Ann. N.Y. Acad. Sci. 107, 878 (1963)];

(f) fusaric acid derivatives and analogues as reported by Runti et al. in Il Farmaco Ed. Sci. 36, 260 (1980). These derivatives include phenylpicolinic acid, which has twice the inhibitory activity of fusaric acid, and 5-(4-chlorobutyl) picolinic acid, and others such as substituted amides of fusaric acid and acids and amides of 5-butyroylpicolinic acid, 5-aminopicolinic acid and 5-hydrazinopicolinic acid, and derivatives thereof;

(g) 5-(3,4-dibromobutyl)picolinic acid and 5-(dimethyldithiocarbamoylmethyl)picolinic acid (Hidaka et al., Molecular Pharmacology 9, 172-177, 1972);

(h) Bupicomide, 5-(n-butyl)picolinamine, Ehrreich et al., "New Antihypertensive Drugs", Spectrum Publications, 1976, pg. 409-432, reported as a DBH inhibitor that has antihypertensive activity;

(i) a series of 1-phenyl and 1-phenylalkylimidazole compositions having a mercapto or alkylthio group in the 2-position European Patent Application No. 125,033 (published November 14, 1984);

(j) Methylpyridine derivatives isolated from the fermentation broth of a strain of Streptoverticillium (United States Patent No. 4,487,761); and

(k) 1-Benzyl-2-aminomethylimidazole derivatives (United States Patent No. 4,532,331).

However, non-specific, often toxic effects to known DBH inhibitors have obviated clinical use of these compounds. Fusaric acid, for example, is hepatotoxic. See, for example, Teresawa et al., Japan. Cir. J. 35, 339 (1971) and references cited therein.

Certain substituted triazoles are known in the literature, for example :

(i) Sci. Phar., 1979, 47(4), 314 (CA 93:46527A) discloses 3-mercapto-4-benzyl-5-(4-pyridyl)-1,2,4-triazole;

(ii) the same reference also discloses, 3-mercapto-4-phenyl-5-(4-pyridyl)-1,2,4-triazole, and anagogues thereof in which the phenyl group is substituted by a 2, 3 or 4-methyl group or a 2-methoxy or 4-chloro group;

(iii) Indian Journal of Chemistry, 1977, 15B(4), 341 (CA 87 : 201478g) discloses 3-mercapto-4-phenyl--5-(3-pyridyl)-1,2,4-triazole, and

(iv) CA 94:156827b, discloses 3-mercapto-4-phenyl-5-(2-pyridyl)-1,2,4-triazole, and the corresponding analogues in which the phenyl ring is substituted by a 4-methyl or 4-bromo group.

However, in all the above-noted references, the compounds are disclosed only as compounds of synthetic interest or as intermediates, no therapeutic activity or utility being noted for them. It has now been found that 4-aralkyl-5-pyridylalkyl-1,2,4-triazoles are also inhibitors of DBH activity, and are useful in the treatment of elevated blood pressure in mammals.

## SUMMARY OF THE INVENTION

The present invention relates to 3-mercapto-4-aralkyl-5-pyridyl-1,2,4-triazoles which have been found to be potent and prolonged inhibitors of DBH.

Presently preferred compounds of the invention include:
3-mercapto-4-benzyl-5-(3-pyridyl)-1,2,4-triazole, and
3-mercapto-4-benzyl-5-(2-pyridyl)-1,2,4-triazole.

In addition the invention relates to pharmaceutical compositions comprising the compounds of the invention and a method of inhibiting DBH activity in mammals, including humans, which comprises administering internally to a subject an effective amount of a compound of the invention.

## DETAILED DESCRIPTION OF THE INVENTION

The present invention relates to compounds of structure (I) :

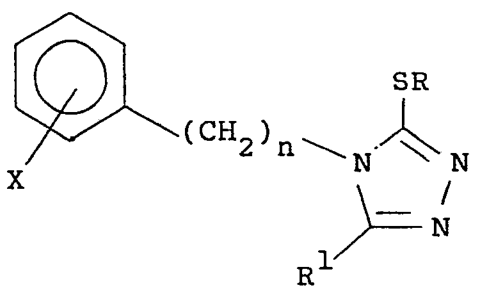

in which

X is hydrogen, halogen, $C_{1-4}$alkyl, $C_{1-4}$alkoxy, OH, CN, $NO_2$, $SO_2NH_2$, $CO_2H$, $CO_2C_{1-4}$alkyl, $CONH_2$, CHO, $CH_2OH$, $C_pF_{2p+1}$, $SO_2C_pH_{2p+1}$ or $SO_2C_pF_{2p+1}$ where p is 1 to 4 or any synthetically acceptable combination thereof up to 5 substituents.

n is 0 to 5;

R is hydrogen or $C_{1-4}$alkyl; and

$R^1$ is $Pyr(CH_2)_m$- in which Pyr is a 2, 3 or 4 pyridyl group and m is 0 to 5, or a pharmaceutically acceptable salt or hydrate thereof, provided that

(i) when R is hydrogen, Pyr is 4-pyridyl, m is 0 and n is 1, X is other than hydrogen;

(ii) when R is hydrogen, Pyr is 4-pyridyl, m and n are both 0, X is other than hydrogen, 2, 3 or 4-$C_{1-4}$alkyl, 2-$C_{1-4}$alkoxy or 4-halogen;

(iii) when R is hydrogen, Pyr is 3-pyridyl, and m and n are both 0, X is other than hydrogen; and

(iv) when R is hydrogen, Pyr is 2-pyridyl, and n and m are both 0, X is other than hydrogen, 4-halogen or 4-$C_{1-4}$alkoxy.

Suitably X is hydrogen, halogen, $C_{1-4}$alkyl, $C_{1-4}$alkoxy, OH, CN, $NO_2$, $SO_2NH_2$, $CO_2H$, $CO_2C_{1-4}$alkyl, $CONH_2$, CHO, $CH_2OH$, $C_pF_{2p+1}$, $SO_2C_pH_{2p+1}$ or $SO_2C_pF_{2p+1}$ where p is 1 to 4 or any synthetically acceptable combination thereof up to 5 substituents; preferably X is a single substituent selected from those defined above, most preferably X is hydrogen;

Suitably, n is 0 to 5, preferably n is 0, 1 or 2; most preferably n is 1.

Suitably m is 0 to 5, preferably m is 0 or 1; most preferably m is 0.

Suitably Pyr is 2-, 3- or 4-pyridyl, preferably Pyr is 2- or 3-pyridyl; most preferably 2-pyridyl.

As used herein, "synthetically accessible combination thereof" means any combination of the substituents that is available by chemical synthesis and is stable.

$C_{1-4}$alkyl ether alone or as part of another group e.g. $C_{1-4}$alkoxy means a straight or branched chain alkyl having from 1 to 4 carbons.

It will be apparent that compounds of structure (I) in which R is hydrogen can exist in a number of tautomeric forms i.e.

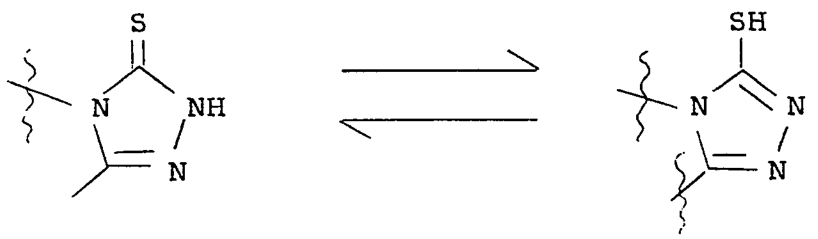

It is intended that structure (I) includes such tautomeric forms.

The compounds of structure (I) can be prepared by processes analogous to those known in the art, in particular compounds of structure (I) can be prepared by reacting a suitable aryl or aralkyl isothiocyanate of structure (II) with a suitable pyridyl hydrazine of structure (III) :

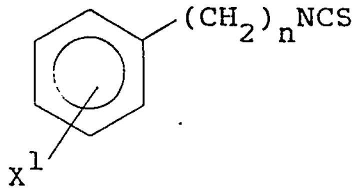

(II)

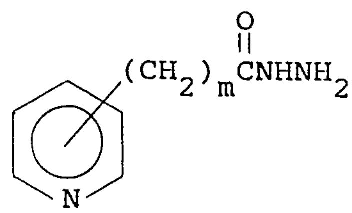

(III)

in which m and n are as described for structure (I) and $X^1$ is X as described for structure (I) but not hydroxy, and optionally thereafter alkylating the compound of structure (I) so formed in which R is hydrogen to form a compound in which R is $C_{1-4}$alkyl and optionally forming a pharmaceutically acceptable salt or hydrate.

The reaction between a compound of structure (II) and a compound of structure (III) is carried out at elevated temperature in a suitable solvent in the presence of a base. In particular, the reaction can be carried out in a $C_{1-4}$alkanol, preferably ethanol, in the presence of a $C_{1-4}$alkali metal oxide, in particular sodium ethoxide, at reflux temperature for a period of up to 24 hours.

The compounds of structure (II) and (III) are commercially available or prepared by standard techniques.

It will be appreciated that

° compounds of structure (I) in which X is hydroxy can be prepared from compounds of structure (I) in which X is $C_{1-4}$alkoxy e.g. methoxy by using known hydrolysis methods for example by treatment with boron tribromide or hydrogen bromide in an appropriate solvent;

° compounds of structure (I) in which R is $C_{1-4}$alkyl can be prepared by alkylating a compound of structure (I) in which R is hydrogen using an appropriate $C_{1-4}$alkylhalide such as methyliodide or butyl bromide with or without a base such as an alkoxide or tertiary amine in a $C_{1-4}$alkanol such as methanol; and

° pharmaceutically acceptable acid addition salts of compounds of structure (I) can be formed with appropriate organic or inorganic acids by methods known in the art. For example, the free base can be reacted with a suitable inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Exemplary of such salts are the maleate, fumarate, lactate, oxalate, methanesulfonate, ethanesulfonate, benzenesulfonate, tartrate, citrate, hydrochloride, hydrobromide, sulfate, phosphate, quinate and nitrate salts.

The compound of structure (IA):

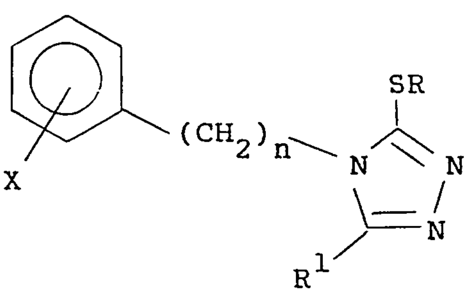

(IA)

in which

X is hydrogen, halogen, $C_{1-4}$alkyl, $C_{1-4}$alkoxy, OH, CN, $NO_2$, $SO_2NH_2$, $CO_2H$, $CO_2C_{1-4}$alkyl, $CONH_2$, CHO, $CH_2OH$, $C_pF_{2p+1}$, $SO_2C_pH_{2p+1}$ or $SO_2C_pF_{2p+1}$ where p is 1 to 4 or any synthetically acceptable combination thereof up to 5 substituents.

n is 0 to 5;

R is hydrogen or $C_{1-4}$alkyl; and
$R^1$ is $Pyr(CH_2)_m$- in which Pyr is a 2, 3 or 4 pyridyl group and m is 0 to 5, or a pharmaceutically acceptable salt or hydrate thereof have been found to be potent and long lasting inhibitors of DBH activity, and as such they are useful as diuretic, natriuretic, cardiotonic, antihypertensive, and vasodilator agents, as well as anti-ulcerogenic and anti-Parkinsonian agents. In a further aspect the present invention therefore provides a method of inhibiting DBH activity in mammals which comprises administering to a subject in need thereof an effective amount of a compound of structure (IA).

Listed in Table I are compounds of the invention that were tested for in vitro DBH inhibition by a standard procedure for assaying conversion of tyramine to octopamine in the presence of DBH. J.J. Pisano, et al., Biochim. Biophys. Acta, 43, 566-568 (1960). Octopamine was assayed following sodium periodate oxidation to p-hydroxybenzaldehyde by measuring spectrophotometric absorbance at 330 nm. In Table I, inhibition is given in molar concentration of compound at which DBH activity was halved ($IC_{50}$). Fusaric acid, by this test has an $IC_{50}$ of 8 x $10^{-7}$M.

Table I

| Compound | DBH $IC_{50}$ (M) |
|---|---|
| 3-mercapto-4-benzyl-5-(2-pyridyl)-1,2,4-triazole | 1.3 x $10^{-5}$ |
| 3-mercapto-4-benzyl-5-(3-pyridyl)-1,2,4-triazole | 5.6 x $10^{-5}$ |
| 3-mercapto-4-benzyl-5-(4-pyridyl)-1,2,4-triazole | 7.0 x $10^{-5}$ |

Further, spontaneously hypertensive rats were treated with 3-mercapto-4-benzyl-5-(4-pyridyl)-1,2,4-triazole at a dose of 100 mg/kg orally and mean arterial blood pressure was monitored for 250 minutes using indwelling cannulae in the tail arteries. When compared to vehicle-treated controls, the animals treated with this compound exhibited significant blood pressure reductions between 80 and 240 minutes following treatment and exhibited their lowest blood pressures when monitoring was discontinued. The maximal blood pressure reduction was approximately 25 mmHg.

When used in the method of the present invention the compounds are incorporated into standard pharmaceutical compositions. In a further aspect the present invention provides pharmaceutical compositions comprising a compound of structure (IA) or a pharmaceutically acceptable salt or hydrate thereof and a pharmaceutical carrier.

The compounds of structure (IA) can be incorporated into convenient pharmaceutical dosage forms such as capsules, tablets, or injectable preparations. Solid or liquid pharmaceutical carriers can be employed. Solid carriers include, starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, along or with a wax. The amount of solid carrier varies widely but, preferably, will be from about 25 mg to about 1 g per dosage unit. When a liquid carrier is used, the preparation will be in the form of a syrup, elixir, emulsion, soft gelatin capsule, sterile injectable liquid, or an aqueous or nonaqueous liquid suspension.

The pharmaceutical preparations are made following conventional techniques of a pharmaceutical chemist involving mixing, granulating and compressing, when necessary, for tablet forms, or mixing, filling, and dissolving the ingredients, as appropriate, to give the desired oral or parenteral products.

Doses of the present compounds of Structure (IA) in a pharmaceutical dosage unit as described above will be an efficacious, non-toxic quantity selected from the range of 0.1-100 mg/kg of active compound, preferably 0.1-50 mg/kg. The selected dose is administered to a human patient in need of DBH inhibition from 1-6 times daily, orally, rectally, by injection, or continuously by infusion. Oral dosage units for human administration preferably contain from 1 to 500 mg of active compound. Parenteral administration, which uses lower dosages is preferred. Oral administration, as higher dosages, however, also can be used when safe and convenient for the patient.

The following examples serve to illustrate the invention.

## Example 1

### 3-mercapto-4-benzyl-5-(2-pyridyl)-1,2,4-triazole

(a) Preparation of picolinic acid hydrazide

A solution of ethyl picolinate (15.1 g, 0.10 mole) and hydrazine monohydrate (7.35 ml, 0.15 mole) in ethanol (150 ml) was heated at reflux for 17 hours then cooled and concentrated to give the product after triturating the residual oil with ether and recrystalising from ethanol- water: melting point 95-97°C (7.78 g, 59%).

(b) Preparation of 3-mercapto-4-benzyl-5-(2-pyridyl)-1,2,4-triazole

A solution of benzyl isothiocyanate (3.32 ml, 0.025 mole), picolinic acid hydrazide (3.43 g, 0.025 mole), and sodium ethoxide [from sodium (1.15 g, 0.05 mole) in ethanol (40 ml)] was heated at reflux for 17 hours then cooled to give the product after removing the ethanol under vacuum, diluting the residue with water and acidifying with 10% hydrochloric acid. The crude product was recrystallised twice from ethanol to give a solid with melting point 185.5-186.5°C (3.24 g, 48%).

## Example 2

3-Mercapto-4-benzyl-5-(3-pyridyl)-1,2,4-triazole

A solution of benzyl isothiocyanate (3.32 ml, 0.025 mole), nicotinic acid hydrazide (3.43 g, 0.025 mole), sodium ethoxide [from sodium (1.15 g, 0.05 mole) in ethanol (40 ml)] was heated at reflux for 17 hours then cooled to give the product after removing the ethanol under vacuum, diluting the residue with water and acidifying with 10% hydrochloric acid. The crude product was recrystallised twice from ethanol to give a solid with melting point 174-175°C (3.73 g, 56%).

## Example 3

3-Mercapto-4-benzyl-5-(4-pyridyl)-1,2,4-triazole

A solution of benzyl isothiocyanate (3.32 ml, 0.025 mole), isonicotinic acid hydrazide (30.43 g, 0.025 mole), sodium ethoxide [from sodium (1.15 g, 0.05 mole) in ethanol (40 ml)] was heated under reflux for 17 hours then cooled to give the product after removing the ethanol under vacuum diluting the residue with water and acidifying with 10% hydrochloric acid. The crude product was recrystallised from ethanol to give a solid with melting point 210-211°C (4.11 g, 61%).

Using the same procedures as hereinabove described, the following compounds may also be prepared.

3-Mercapto-4-[3-fluorobenzyl]-5-(4-pyridyl)-1,2,4-triazole

The reaction of 3-fluorobenzylisothiocyanate, isonicotinic acid hydrazide and sodium ethoxide yields the title compound.

3-Mercapto-4-[3-5-difluorobenzyl]-5-(4-pyridyl)-1,2,4-triazole

The reaction of 3-5-difluorobenzylisothiocyanate, isonicotinic acid hydrazide and sodium ethoxide yields the title compound.

3-Mercapto-4-[3-5-difluorophenylpropyl]-5-(4-pyridyl)-1,2,4-triazole

The reaction of 3-5-difluorophenylpropylisothiocyanate, isonicotinic acid hydrazide and sodium ethoxide yields the title compound.

3-Mercapto-4-[3-chlorobenzyl]-5-(4-pyridyl)-1,2,4-triazole

The reaction of 3-chlorobenzylisothiocyanate, isonicotinic acid hydrazide and sodium ethoxide yields the title compound.

3-Mercapto-4-[4-methoxybenzyl]-5-(3-pyridyl)-1,2,4-triazole

The reaction of 4-methoxybenzylisothiocyanate, nicotinic acid hydrazide and sodium ethoxide yields the title compound.

3-Mercapto-4-[4-hydroxybenzyl]-5-(3-pyridyl)-1,2,4-triazole

The reaction of 3-mercapto-4-[4-methoxybenzyl]-5-[3-pyridyl]-1,2,4-triazole with 3 equiv. of boron tribromide in dichloromethane at 0°C followed by aqueous workup yields the title compound.

3-Mercapto-4-[phenethyl]-5-(2-pyridyl)-1,2,4-triazole

The reaction of phenethylisothiocyanate, picolinic acid hydrazide and sodium ethoxide yields the title compound.

3-Methylthio-4-[benzyl]-5-(2-pyridyl)-1,2,4-triazole

The reaction of 3-mercapto-4-[benzyl]-5-[2-pyridyl]-1,2,4-triazole with 1 equivalent of methyliodide and 1 equivalent of sodium methoxide in methanol yields the title compound.

3-Butylthio-4-[benzyl]-5-(2-pyridyl)-1,2,4-triazole

The substitution of n-butyl iodide for methyl iodide in the previous example yields the title compound.

3-Mercapto-4-[3-trifluoromethylbenzyl]-5-(2-pyridyl)-1,2,4-triazole

The reaction of 3-trifluoromethylbenzylisothiocyanate, picolinic acid hydrazide and sodium ethoxide yields the title compound.

## Example 4

An oral dosage form for administering the presently invention compounds is produced by screening, mixing, and filling into hard gelatin capsules the ingredients in the proportions shown in Table II, below:

Table II

| Compound | Amounts |
|---|---|
| 3-mercapto-4-benzyl-5-(2-pyridyl)-1,2,4-triazole | 50 mg |
| magnesium stearate | 5 mg |
| lactose | 75 mg |

## Example 6

The sucrose, calcium sulfate dihydrate, and compound shown in Table III below, are mixed and granulated in the proportions shown with a 10°% gelatin solution. The wet granules are screened, dried, mixed with the starch, talc and stearic acid, screened and compressed into a tablet.

Table III

| Compound | Amounts |
|---|---|
| 3-mercapto-4-benzyl-5-(2-pyridyl)-1,2,4-triazole | 100 mg |
| calcium sulfate dihydrate | 150 mg |
| sucrose | 20 mg |
| starch | 10 mg |
| talc | 5 mg |
| stearic acid | 3 mg |

## Example 7

3-Mercapto-4-benzyl-5-(2-pyridyl)-1,2,4-triazole is dispersed in 25 ml of normal saline to prepare an injectable preparation.

## Claims

1. A compound of formula (I)

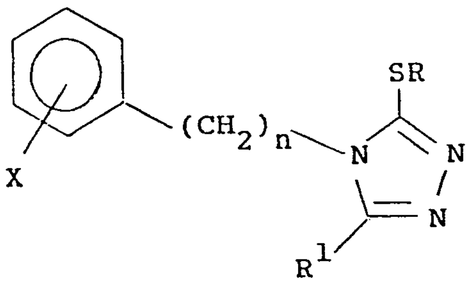

(I)

in which

X is hydrogen, halogen, $C_{1-4}$alkyl, $C_{1-4}$alkoxy, OH, CN, $NO_2$, $SO_2NH_2$, $CO_2H$, $CO_2C_{1-4}$alkyl, $CONH_2$, CHO, $CH_2OH$, $C_pF_{2p+1}$, $SO_2C_pH_{2p+1}$ or $SO_2C_pF_{2p+1}$ where p is 1 to 4 or any synthetically acceptable combination thereof up to 5 substituents.

n is 0 to 5;

R is hydrogen or $C_{1-4}$alkyl; and

$R^1$ is $Pyr(CH_2)_m$- in which Pyr is a 2, 3 or 4 pyridyl group and m is 0 to 5, or a pharmaceutically acceptable salt or hydrate thereof, provided that

(i) when R is hydrogen, Pyr is 4-pyridyl, m is 0 and n is 1, X is other than hydrogen;

(ii) when R is hydrogen, Pyr is 4-pyridyl, m and n are both 0, X is other than hydrogen, 2, 3 or 4-$C_{1-4}$alkyl, 2-$C_{1-4}$alkoxy or 4-halogen;

(iii) when R is hydrogen, Pyr is 3-pyridyl, and m and n are both 0, X is other than hydrogen; and

(iv) when R is hydrogen, Pyr is 2-pyridyl, and n and m are both 0, X is other than hydrogen, 4-halogen or 4-$C_{1-4}$alkoxy.

2. A compound as claimed in claim 1 which is 3-mercapto-4-benzyl-5-(3-pyridyl)-1,2,4-triazole.

3. A compound as claimed in claim 1 which is 3-mercapto-4-benzyl-5-(2-pyridyl)-1,2,4-triazole.

4. A pharmaceutical composition comprising a compound of structure (IA):

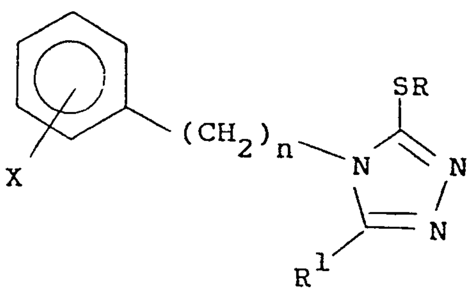

(IA)

in which

X is hydrogen, halogen, $C_{1-4}$alkyl, $C_{1-4}$alkoxy, OH, CN, $NO_2$, $SO_2NH_2$, $CO_2H$, $CO_2C_{1-4}$alkyl, $CONH_2$, CHO, $CH_2OH$, $C_pF_{2p+1}$, $SO_2C_pH_{2p+1}$ or $SO_2C_pF_{2p+1}$ where p is 1 to 4 or any synthetically acceptable combination thereof up to 5 substituents.

n is 0 to 5;

R is hydrogen or $C_{1-4}$alkyl; and

$R^1$ is $Pyr(CH_2)_m$- in which Pyr is a 2, 3 or 4 pyridyl group and m is 0 to 5,

or a pharmaceutically acceptable salt or hydrate thereof;

and a pharmaceutically acceptable carrier.

5. A pharmaceutical composition as claimed in claim 4 in which the compound of structure (IA) is

3-mercapto-4-benzyl-5-(3-pyridyl)-1,2,4-triazole,

3-mercapto-4-benzyl-5-(2-pyridyl)-1,2,4-triazole or

3-mercapto-4-benzyl-5-(4-pyridyl)-1,2,4-triazole.

6. A compound according to any of claims 1 to 3 for use as a medicament.

7. A process for preparing a pharmaceutical composition which comprises bringing into association a compound of formula (IA) or a pharmaceutically acceptable salt thereof as defined in claim 4 and a pharmaceutically acceptable carrier.

8. The use of a compound of formula (IA) or a pharmaceutically acceptable salt thereof as defined in claim 4 in the manufacture of a medicament for inhibiting dopamine-β-hydroxylase.

9. The use of a compound of structure (IA) or a pharmaceutically accepted salt thereof as defined in claim 4 for reducing blood pressure.

**Claims for the following Contracting States: ES,GR**

1. A process for preparing a compound of formula (I)

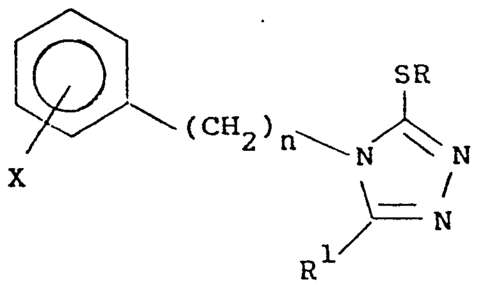

(I)

in which

X is hydrogen, halogen, $C_{1-4}$alkyl, $C_{1-4}$alkoxy, OH, CN, $NO_2$, $SO_2NH_2$, $CO_2H$, $CO_2C_{1-4}$alkyl, $CONH_2$, CHO, $CH_2OH$, $C_pF_{2p+1}$, $SO_2C_pH_{2p+1}$ or $SO_2C_pF_{2p+1}$ where p is 1 to 4 or any synthetically acceptable combination thereof up to 5 substituents.

n is 0 to 5;

R is hydrogen or $C_{1-4}$alkyl; and

$R^1$ is Pyr$(CH_2)_m$- in which Pytr is a 2, 3 or 4 pyridyl group and m is 0 to 5, or a pharmaceutically acceptable salt or hydrate thereof, provided that

(i) when R is hydrogen, Pyr is 4-pyridyl, m is 0 and n is 1, X is other than hydrogen;

(ii) when R is hydrogen, Pyr is 4-pyridyl, m and n are both 0, X is other than hydrogen, 2, 3 or 4-$C_{1-4}$alkyl, 2-$C_{1-4}$alkoxy or 4-halogen;

(iii) when R is hydrogen, Pyr is 3-pyridyl, and m and n are both 0, X is other than hydrogen; and

(iv) when R is hydrogen, Pyr is 2-pyridyl, and n and m are both 0, X is other than hydrogen, 4-halogen or 4-$C_{1-4}$alkoxy.

which process comprises reacting a compound of formula (II)

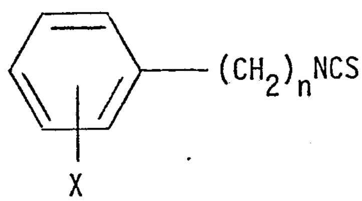

(II)

wherein X and n are as defined above,

with a compound of formula (III)

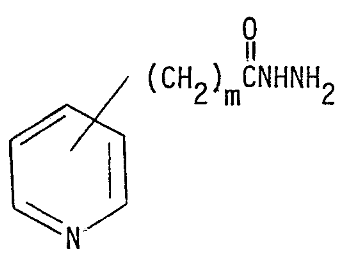

(III)

wherein m is as defined above,

and thereafter, if R is $C_{1-4}$alkyl, alkylating the product with a $C_{1-4}$alkylating agent.

2. A process according to Claim 1 for preparing 3-mercapto-4-benzyl-5-(2-pyridyl)-1,2,4-triazole or 3-mercapto-4-benzyl-5-(3-pyridyl)-1,2,4-triazole.

3. A process for preparing a pharmaceutical composition which comprises bringing into association a compound of formula (I) or a pharmaceutically acceptable salt thereof as defined in Claim 1 and a pharmaceutically acceptable carrier.

4. A process according to Claim 3 in which the compound of formula (I) is selected from 3-mercapto-4-benzyl-5-(2-pyridyl)-1,2,4-triazole or 3-mercapto-4-benzyl-5-(3-pyridyl)-1,2,4-triazole.

European Patent Office

# PARTIAL EUROPEAN SEARCH REPORT

which under Rule 45 of the European Patent Convention shall be considered, for the purposes of subsequent proceedings, as the European search report

Application number: EP 89309202.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | CHEMICAL ABSTRACTS, vol. 87, no. 25, December 19, 1977, Columbus, Ohio, USA NATH, T.G. SURENDRA et al. "Synthesis and reactivity of 4,5-disubstituted 3--chloro-1,2,4-triazoles and their methylsulfonyl analogs" page 724, column 2, Abstract--no. 201 478g & Indian J. Chem., Sect. B 1977, 15B(4), 341-6 -- | 1 | C 07 D 401/06<br>A 61 K 31/44 |
| D,A | CHEMICAL ABSTRACTS, vol. 94, no. 19, May 11, 1981, Columbus, Ohio, USA BANY, TADEUSZ et al. "Synthesis of some derivatives of 1,2,4-triazole and 1,3,4--thiadiazole ring systems using N3-substituted amidrazones as starting products" page 651, column 2, Abstract- | 1 | |

TECHNICAL FIELDS SEARCHED (Int. Cl.4)

C 07 D 401/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-8

Claims searched incompletely: –

Claims not searched: 9

Reason for the limitation of the search:

(Art. 52 (4) EPC; method for treatment of the human or animal body by therapy)

| Place of search | Date of completion of the search | Examiner |
|---|---|---|
| VIENNA | 23-11-1989 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| | -no. 156 827b<br>& Ann. Univ. Mariae Curie-<br>-Sklodowska, Sect. AA: Chem.<br>1976-1977 (Pub. 1980). 31-32,<br>277-83<br>-- | | |
| D,A | CHEMICAL ABSTRACTS, vol. 93,<br>no. 5, August 4, 1980,<br>Columbus, Ohio, USA<br>EL-KHAWASS, S.M. et al.<br>"Synthesis of 3,4-disubstitu-<br>ted-5-carboxy/carbethoxy-<br>methylthio-1,2,4-(4H)tria-<br>zoles, as potential anti-<br>microbial agents"<br>page 920, column 1, Abstract-<br>-no. 46 527a<br>& Sci. Pharm. 1979, 47(4),<br>314-19<br>-- | 1 | TECHNICAL FIELDS SEARCHED (Int. Cl.4) |
| A | CHEMICAL ABSTRACTS, vol. 71,<br>no. 7, August 18, 1969,<br>Columbus, Ohio, USA<br>KREYSIG, DIETER et al.<br>"Heterocycles from amino<br>ketones. XV. (Aminooxadia-<br>zolyl)- and (thioxotriazo-<br>linyl)quinolylmethanes"<br>page 290, column 1, Abstract-<br>-no. 30 411t<br>& Z. Chem. 1969, 9(5), 187-8<br>-- | 1 | |
| A | CHEMICAL ABSTRACTS, vol. 67,<br>no. 7, August 14, 1967,<br>Columbus, Ohio, USA<br>M.DZIEWONSKA "Infrared<br>spectra of some 3,4,5-<br>-substituted derivatives of<br>1,2,4-triazole"<br>page 3564, column 2,<br>Abstract-no. 37 867x<br>& Spectrochim. Acta, Part A<br>23(5), 1195-204(1967)<br>-- | 1 | |
| D,A | US - A - 4 532 331<br>(FRAZEE) | 4-8 | |

European Patent Office

# PARTIAL EUROPEAN SEARCH REPORT

Application number
-3-
EP 89309202.3

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| | * Column 5, line 48 - column 7, line 62 *<br>---- | | |
| | | | TECHNICAL FIELDS SEARCHED (Int. Cl.4) |